# EUROPEAN PATENT APPLICATION

(11) **EP 4 473 964 A1**
(43) Date of publication of application: **11.12.2024**
(21) Application number: 23749915.7
(22) Date of filing: 16.01.2023
(51) Int. Cl.: A61K 31/353, A61K 31/445, A61P 25/28, A23L 33/10

(54) **COMPOSITION COMPRISING BETA BLOCKER AND CHOLINESTERASE INHIBITOR FOR TREATMENT OF NEURODEGENERATIVE DISEASE**

(30) Priority: 04.02.2022 KR 20220014784
(71) Applicant: Dr.Noah Biotech Inc., Suwon-si, Gyeonggi-do 16229 (KR)
(72) Inventor: LEE, Ji Hyun, Suwon-si Gyeonggi-do 16229 (KR); KIM, Eun Jung, Suwon-si Gyeonggi-do 16229 (KR)
(74) Representative: Schwarz & Partner Patentanwälte GmbH
(86) International application number: PCT/KR2023/000760
(87) International publication number: WO 2023/149682

(57) **Abstract**

The present invention relates to a composition for treating neurodegenerative diseases comprising a beta blocker and a cholinesterase inhibitor and, more specifically, to a composition that relieves neuroinflammation, promotes nerve cell differentiation and shows antioxidant effects to exhibit synergistic effects on degenerative neurological diseases.

## Description

### Technical Field

The present application claims priority to Korean Patent Application No. 2022-0014784 filed on February 4, 2022, and the entire specification thereof is incorporated herein by reference.

The present invention relates to a composition for treating neurodegenerative diseases comprising a beta blocker and a cholinesterase inhibitor and, more specifically, to a composition that relieves neuroinflammation, promotes nerve cell differentiation and shows antioxidant effects to exhibit synergistic effects on degenerative neurological diseases.

### Background Art

A neurodegenerative disease refers to a disease that occurs in the brain or spinal cord among degenerative diseases that occur with aging, and is caused by gradual loss of the structure and function of certain brain cell populations in the brain and spinal cord due to unknown causes, genetic defects or environmental factors. These neurodegenerative diseases comprise Alzheimer's disease (AD), Parkinson's disease (PD), Huntington's disease (HD), multiple sclerosis (MS) and amyotrophic lateral sclerosis (ALS) also known as Lou Gehrig's disease.

Known causes of Alzheimer's disease are reduction in neurotransmission due to a decrease in acetylcholine concentration, occurrence of neuroinflammation due to cytokines, cytotoxicity due to aggregation of amyloid-beta and hyperphosphorylated tau protein, and neuronal cell death by reactive oxygen species caused by oxidative stress. Since such neuronal cell death is pointed out as a fundamental pathology common to various brain diseases, in-depth studies on factors and molecular mechanisms that cause apoptosis are being conducted. However, the previously developed degradative enzymes or broad-spectrum apoptosis inhibitors have side effects and reduced clinical effectiveness.

ALS is a fatal neurodegenerative disease that motor neurons are selectively lost by apoptosis in the spinal cord. About 10 to 20% of patients show a genetic pattern (familial ALS (fALS)), and the rest are classified as sporadic ALS (sALS) . A part of genes are known in the ALS-associated genetic loci of familial ALS, among which SOD1 is the first gene identified in fALS. Although fALS-related genes are expected to play a role in the pathogenesis of sALS, the exact cause of sALS has not been identified so far. In addition, ALS is classified into typical ALS, ALS with dementia, and atypical ALS according to clinical symptoms. Indeed, mutations in SOD1 cause classic ALS, and C9orf72 is associated with ALS with dementia.

One of the important features of ALS is that it is a progressive disease, in which neuronal cell death propagates through neural connections. In fact, Alzheimer's and Parkinson's diseases show similar phenotypes. In connection with such feature, the prion-like propagation mechanism that misfolded proteins transform normal proteins into abnormal ones has been raised. Indeed, mutant Amyloid beta (Aβ) may transform normal Aβ into abnormal Aβ. Recently, it has been reported that mutated or misfolded SOD1 may also be secreted and disseminated during disease progression. However, not much research has been conducted on SOD1 aggregation and misfolding inhibitors targeting ALS disease therapeutics.

### Summary of Invention

### Technical Problem

Accordingly, the inventors of the present invention conducted extensive research to develop a therapeutic combination that exhibits a synergistic effect in the treatment of neurodegenerative diseases among known substances whose safety has been clinically confirmed, and confirmed that a combined formulation comprising a beta blocker and a cholinesterase inhibitor exhibited significantly improved effects such as alleviating neuroinflammation and promoting neuronal differentiation, as compared to each drug alone, and have completed the present invention.

An aspect of the present invention is to provide a pharmaceutical composition comprising a beta blocker and a cholinesterase inhibitor as active ingredients for preventing or treating a neurodegenerative disease.

Still, another aspect of the present invention is to provide a pharmaceutical composition consisting of a beta blocker and a cholinesterase inhibitor as active ingredients for preventing or treating a neurodegenerative disease.

Still, another aspect of the present invention is to provide a pharmaceutical composition essentially consisting of a beta blocker and a cholinesterase inhibitor as active ingredients for preventing or treating a neurodegenerative disease.

Still, another aspect of the present invention is to provide a food composition comprising a beta blocker and a cholinesterase inhibitor for preventing or alleviating a neurodegenerative disease.

Still, another aspect of the present invention is to provide a food composition consisting of a beta blocker and a cholinesterase inhibitor for preventing or alleviating a neurodegenerative disease.

Still, another aspect of the present invention is to provide a food composition essentially consisting of a beta blocker and a cholinesterase inhibitor for preventing or alleviating a neurodegenerative disease.

Still, another aspect of the present invention is to provide use of a beta blocker and a cholinesterase inhibitor for the preparation of an agent for preventing or treating a neurodegenerative disease.

Still, another aspect of the present invention is to provide a method for treating a neurodegenerative disease in a subject in need thereof, the method comprising administering an effective amount of a composition comprising a beta blocker and a cholinesterase inhibitor as active ingredients.

### Solution to Problem

Accordingly, in accordance with the aspect of the present invention, there is provided a pharmaceutical composition comprising a beta blocker and a cholinesterase inhibitor as active ingredients for preventing or treating a neurodegenerative disease.

In accordance with another aspect of the present invention, there is provided a pharmaceutical composition consisting of a beta blocker and a cholinesterase inhibitor as active ingredients for preventing or treating a neurodegenerative disease.

In accordance with another aspect of the present invention, there is provided a pharmaceutical composition essentially consisting of a beta blocker and a cholinesterase inhibitor as active ingredients for preventing or treating a neurodegenerative disease.

In accordance with another aspect of the present invention, there is provided a food composition comprising a beta blocker and a cholinesterase inhibitor for preventing or alleviating a neurodegenerative disease.

In accordance with another aspect of the present invention, there is provided a food composition consisting of a beta blocker and a cholinesterase inhibitor for preventing or alleviating a neurodegenerative disease.

In accordance with another aspect of the present invention, there is provided a food composition essentially consisting of a beta blocker and a cholinesterase inhibitor for preventing or alleviating a neurodegenerative disease.

In accordance with another aspect of the present invention, there is provided use of a beta blocker and a cholinesterase inhibitor for the preparation of an agent for preventing or treating a neurodegenerative disease.

In accordance with another aspect of the present invention, there is provided a method for treating a neurodegenerative disease in a subject in need thereof, the method comprising administering an effective amount of a composition comprising a beta blocker and a cholinesterase inhibitor as active ingredients.

As used herein, the term "treatment" or "treating" refers to application or administration of a therapeutic agent, i.e., a compound of the present invention (either alone or in combination with other pharmaceutical agents), to a patient, or application or administration of the therapeutic agent to an isolated tissue or cell line for the purpose of treating, curing, relieving, alleviating, altering, solving or improving a patient's HBV infection, symptoms of HBV infection, or potential to develop HBV infection (e.g., diagnostic and disembodied application), or affecting the HBV infection, symptoms of HBV infection, or potential to develop HBV infection. Such treatment may be applied and modified based on knowledge particularly obtained from the field of genomic pharmacology.

As used herein, the term "prevent" or "prevention" refers to no disability or disease progression, if nothing has happened, or no further disability or disease progression if there has already been a disability or disease progression. In addition, contemplated is one's ability to prevent some or all of the symptoms associated with a disorder or disease.

As used herein, the term "patient," "individual" or "subject" refers to a human being or a mammal excluding a human being. For example, a mammal excluding a human being comprises livestock and pets (e.g., ovine, bovine, porcine, canine, feline and murine mammals). Preferably, the patient, subject or individual is a human being.

As used herein, the terms "effective amount," "pharmaceutically effective amount" and "therapeutically effective amount" refer to a non-toxic sufficient amount of an agent to provide a desired biological result. The result may be a reduction and/or attenuation of a signal, symptom or cause of a disease, or any other desired change in a biological system. An appropriate therapeutic amount for any individual may be determined by one skilled in the art using routine experimentation.

As used herein, the term "pharmaceutically acceptable" refers to a substance that does not interfere with the biological activity or properties of a compound and is relatively non-toxic, i.e., a substance such as a carrier or a diluent that may be administered to a subject, without producing undesirable biological effects or interacting in a detrimental way with any component of the composition the substance contains.

As used herein, the term "pharmaceutically acceptable salt" refers to a salt of a compound prepared and administered from non-toxic acids comprising pharmaceutically acceptable inorganic acids, organic acids, solvates, hydrates or clathrates thereof.

Examples of such inorganic acids comprise hydrochloric acid, hydrobromic acid, iodic acid, nitric acid, sulfuric acid, phosphoric acid, acetic acid, hexafluorophosphoric acid, citric acid, gluconic acid, benzoic acid, propionic acid, butyric acid, sulfosalicylic acid, maleic acid, lauric acid, malic acid, fumaric acid, succinic acid, tartaric acid, amsonic acid, pamic acid, p-toluenesulfonic acid and mesylic acid. Suitable organic acids may, for example, be selected from the aliphatic, aromatic, carboxylic and sulfonic classes of organic acids, and, for example, comprises formic acid, acetic acid, propionic acid, succinic acid, camphorsulfonic acid, citric acid, fumaric acid, gluconic acid, isethionic acid, lactic acid, malic acid, mucoic acid, tartaric acid, para-toluenesulfonic acid, glycolic acid, glucuronic acid, maleate acid, furoic acid, glutamic acid, benzoic acid, atranilic acid, salicylic acid, phenylacetic acid, mandelic acid, emphonic acid (pam acid), methanesulfonic acid, ethanesulfonic acid, pantothenic acid, benzenesulfonic acid (besylate), stearic acid, sulfanilic acid, alginic acid and galacturonic acid. In addition, pharmaceutically acceptable salts comprise alkaline earth metal salts (e.g. calcium or magnesium), alkali metal salts (e.g. sodium-dependent and potassium) and ammonium salts, but the present invention is not limited thereto.

As used herein, the term "pharmaceutically acceptable carrier" refers to a pharmaceutically acceptable substance, composition or carrier, for example, liquid or solid fillers, stabilizers, dispersants, suspending agents, diluents, additives, thickening agents, solvents or encapsulating substances, which are involved in the delivery or transport of compounds useful within the present invention so that they can perform their intended function in or to a patient. Typically, these components are carried or transported from one organ or part of the body to another organ or part of the body. Each carrier must be compatible with the other ingredients of the formula comprising compounds useful within the present invention and must be "acceptable" in the sense of not injurious to the patient. Some examples of materials that can serve as pharmaceutically acceptable carriers comprise: sugars such as lactose, glucose and sucrose used in pharmaceutical formulations; starches such as corn starch and potato starch; cellulose and its derivatives such as sodium carboxymethyl cellulose, ethylcellulose and cellulose acetate; tragacanth powder; malt; gelatin; talc; additives such as cocoa butter and suppository wax; oils such as peanut oil, cottonseed oil, safflower oil, sesame oil, olive oil, corn oil and soybean oil; glycols such as propylene glycol; polyols such as glycerin, sorbitol, mannitol and polyethylene glycol; esters such as ethyl oleate and ethyl laurate; agar; buffers such as magnesium hydroxide and aluminum hydroxide; surfactants; alginic acid; pyrogen-free water; isotonic solution; Ringer's solution; ethyl alcohol; phosphate buffer solution; and other non-toxic compatible substances. In addition, as used in the present invention, a "pharmaceutically acceptable carrier" comprises a part or all of a coating agent, an antiviral and antibacterial agent and an absorption delaying agent that is compatible with the activity of the compound useful within the present invention and physiologically acceptable to the patient. In addition, a supplementary active compound may also be incorporated into the composition. In addition, a "pharmaceutically acceptable carrier" may further comprise a pharmaceutically acceptable salt of a compound useful within the present invention. Other additional ingredients that may be contained in the pharmaceutical composition used to practice the present invention are known in the art.

As used herein, the term "combination therapy" or "in combination" refers to the administration of two or more therapeutic agents to treat the conditions or disorders described herein (i.e., metabolic diseases and fibrotic diseases). Such administration comprises administration of these therapeutic agents in a substantially simultaneous manner, such as co-administration in a single capsule having a fixed proportion of the active ingredients. Alternatively, such administration comprises co-administration in multiple or separate containers (e.g., capsules, powders and liquids) for each active ingredient. Powders and/or liquids may be reconstituted or diluted to the desired dosage prior to administration. In addition, such administration comprises sequential use of each type of therapeutic agent almost simultaneously or at different times. In either case, the treatment regimen will provide beneficial effects of the drug combination in the treatment of the conditions or disorders described herein.

Combination therapy can provide a "synergistic effect" and can prove to be "synergistic." That is, the effect achieved when the active ingredients are used together is greater than the sum of the effects produced by using the compounds individually. A synergistic effect can be achieved when the active ingredients are (1) co-formulated and administered or delivered simultaneously in a combined unit dosage form; (2) delivered alternately or in parallel as separate dosage forms; or (3) delivered by some other initiative. When delivered in alternating therapy, a synergistic effect can be obtained when the compounds are administered or delivered sequentially, e.g. by different injections in separate syringes. Generally, during alternating therapy, effective doses of each active ingredient are administered sequentially, i.e., consecutively, whereas in combination therapy, effective dosages of two or more active ingredients are administered together. Synergistic effect as used herein refers to the action of two therapeutic agents to produce an effect greater than the simple sum of the effects of each drug administered alone. The synergistic effect may be calculated using suitable methods such as, for example, the Sigmoid-Emax equation and the Loewe additivity equation. Each of the equations mentioned above may be applied to experimental data to generate corresponding graphs that help evaluate the effects of drug combinations. The corresponding graphs related to the above-mentioned equations are concentration-effect curves, isobologram curves, and combination index curves, respectively.

Hereinafter, the present invention will be described in detail.

An aspect of the present invention is to provide a pharmaceutical composition comprising a beta blocker and a cholinesterase inhibitor as active ingredients for preventing or treating a neurodegenerative disease.

In the present invention, the "beta blocker" refers to a beta-receptor blocking agent, a beta-adrenoceptor blocking agent, a beta-blocking agent, a beta-blocking agent or a beta-adrenoceptor blocking agent, or a natural or artificial compound that inhibits the binding of agonists to all types of beta-adrenergic receptors (beta-1, beta-2, beta-3 or others).

Beta blockers are known to exert a positive effect on the cardiovascular system mainly by blocking cardioselective β1-receptors. A number of different beta blockers have been approved for the treatment of human cardiovascular disease. Due to their inotrope and chronotrop inhibitory effects, beta-blockers directly improve hemodynamic cardiac workload economy. Beta blockers are used in a human being for the treatment of stable chronic heart failure with limited systolic function, arrhythmia, and hyperkinetic heart syndrome; and for the treatment of high blood pressure and coronary artery disease (CAD) and the prevention of heart attack.

In the present invention, the beta blocker may be selected from the group consisting of Nebivolol, Acebutolol, Alprenolol, Atenolol, Betaxolol, Bisoprolol, Bucindolol, Carteolol, Carvedilol, Celiprolol, Esmolol, Labetalol, Levobunolol, Medroxalol, Mepindolol, Metipranolol, Metoprolol, Nadolol, Oxprenolol, Penbutolol, Pindolol, Propranolol, Sotalol and Timolol, but the present invention is not limited thereto. Most preferably, the beta blocker may be nebivolol.

Nebivolol is a compound having the structure of formula 1 below and has beta-blocking properties, but differs from other classical β-blockers in that it has high selectivity for β1 adrenergic receptors and produces vasodilatory effects related to effects on endothelial nitric oxide. Nebivolol is thought to increase the concentration of nitric oxide through the L-arginine-nitric oxide pathway in the vascular endothelium and has been shown to improve endothelial dysfunction and vascular elasticity. Nebivolol has been shown to be beneficial in the treatment of cardiovascular diseases such as hypertension, congestive heart failure, arterial stiffness and endothelial dysfunction.

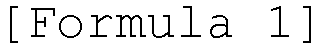

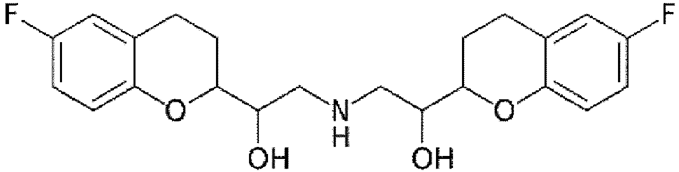

In the present invention, the "cholinesterase inhibitor" refers to a compound that inhibits enzymatic degredation of the neurotransmitter acetylcholine and thus increases the duration and level of action of acetylcholine in the synaptic cleft. Two enzymes are primarily responsible for the breakdown of acetylcholine, acetylcholinesterase and butyrylcholinesterase. The cholinesterase inhibitors comprise substances that inhibit or otherwise reduce the action of one or both of these enzymes.

In the compositions and methods of the present invention, cholinesterase inhibitors are considered pharmaceutically effective. As used herein, 'pharmaceutically effective' indicates that the cholinesterase inhibitor is therapeutically useful in a human being. For this reason, the term excludes cholinesterase inhibitors used as pesticides, such as aldicarb (2-methyl-2-(methylthio)propionaldehyde O-methylcarbamoyloxime), carbofuran (2,3-dihydro-2,2-dimethyl-7-benzofuranyl methylcarbamate), and carbaryl (1-naphthyl methylcarbamate), and cholinesterase inhibitors lethal enough to human beings to be used as chemical weapons, such as sarin (2-(fluoro-methylphosphoryl)oxypropane), VX (S-[2-(diiso) propylamino)ethyl]-O-ethyl methylphosphonothioate), and soman (3-(fluoro-methyl-phosphoryl)oxy-2,2-dimethyl-butane). Most cholinesterase inhibitors as pesticides and chemical weapons are quasi-reversible or irreversible. Most pharmaceutically effective cholinesterase inhibitors are reversible.

Pharmaceutically effective cholinesterase inhibitors are well known in the art. Examples thereof may comprise 7-methoxytacrine, albameline, ambenonium, anseculin, arecoline, cevimeline, citicoline, demacarium, donepizil, edrophonium, eptastigmine, fasciculin, heptyl-physostigmine, huperzine A and its analogs, icopezil, ipidacrine, linopiridine, metrifonate, milameline, neostigmine, nomeostigmine, pyridostigmine, norpyridostigmine, tacrine, physostigmine, rivastigmine, subcomeline, suronacrine, tacrine analogs, tacrine, talsaclidine, velnacrine, xanomeline, ziprosilone, itopride, acotiamide , huperzine, galantamine and salts thereof, but the present invention is not limited thereto. Most preferably, the cholinesterase inhibitor may be donepezil.

Donepezil is an acetylcholinesterase (AChE) inhibitor having a structure of Formula 2 below and is used for the treatment of mild to moderate dementia in Alzheimer's disease. In Alzheimer's disease, in which cholinergic nervous system disorders have been reported in the brain, donepezil activates cholinergic neurons in the brain by increasing acetylcholine in the brain. The currently commercially used formulations of donepezil are in the form of tablets (pills) and are prescribed to patients with Alzheimer's disease in oral form.

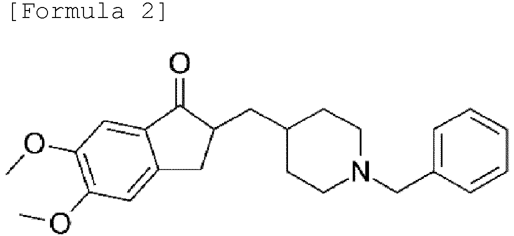

According to one embodiment of the present invention, the composite composition containing nebivolol and donepezil was confirmed to exhibit a synergistic effect in relieving neuroinflammation, neuronal cell differentiation and anti-oxidization, as compared to each formulation alone, such that the composition is effective in preventing or treating neurodegenerative diseases.

Therefore, as compared to using each drug alone, the composition of the present invention comprising a beta blocker and a cholinesterase inhibitor can exhibit a more improved effect with a lower dose such that the composition has the advantages of reducing side effects are reduced and improving brain disease treatment effect, i.e. synergic effect.

The Bliss independence combined response C for two single compounds with effects A and B is C (C = A + B - A*B), where each effect is expressed as a fractional inhibition from 0 to 1 (see Bliss (1939) Annals of Applied Biology) . The Bliss value, defined as the difference between the experimental response and the calculated independent Bliss value, indicates whether the effect of the two components in combination is additive or synergistic.

A bliss value of zero (0) is considered additive. The term "additive" means that the result of combining two types of target agents is the sum of each of the individual drugs.

The term "synergy" or "synergistic" is used to mean that the response of the combination of the two agents is greater than the sum of the responses of the individual agents. More particularly, in an *in vitro* setting, one measure of synergism is known as "Bliss synergy. " The Bliss synergistic effect means "exceeding the Bliss independent value" determined by the previously defined Bliss value. A bliss value greater than zero is considered an indicator of synergy. Of course, the concept of "synergy" as used in the present invention comprises *in vitro* synergy measured by additive and/or alternative methods.

In the present invention, the *in vitro* biological effect (which comprises anti-inflammatory effect, but the present invention is not limited thereto) of the beta blocker and cholinesterase inhibitor combination which is greater than or equal to the sum of the individual components of the combination may be correlated to a bliss value. In addition, as used herein, "a synergistic effect," which comprises cases where a combination of components exhibits an activity equal to or greater than the sum of the individual components, may be measured by additional and/or alternative methods.

In one aspect of the present invention, the composition of the present invention is for treating neurodegenerative diseases, and, in the composition, an effective amount of a beta blocker or a pharmaceutically effective salt, derivative or metabolite thereof may be combined with an effective amount of a cholinesterase inhibitor or pharmaceutically effective salt thereof in an amount sufficient to achieve a synergistic effect.

In one aspect of the present invention, the beta blocker and cholinesterase inhibitor may be contained in a molar ratio of 1:0.1 to 20, preferably 1:0.1 to 10, more preferably 0.1 to 5, even more preferably in a molar ratio of 1:0.1 to 3, much more preferably in a molar ratio of 1:0.3 to 3, and most preferably in a molar ratio of 1:0.5 to 2.

In the present invention, the neurodegenerative disease may be selected from the group consisting of Alzheimer's disease, Parkinson's disease, dementia, cognitive dysfunction, progressive supranuclear palsy, multiple system atrophy, olive-ponytic-cerebellar atrophy (OPCA), Shy-Drager syndrome, striatal-substantia nigra degeneration, Huntington's disease, amyotrophic lateral sclerosis (ALS), essential tremor, cortico-basal ganglia, diffuse Lewy body disease, Parkinson-ALS-dementia complex, Niemann-Pick disease and Pick's disease, but the present invention is not limited thereto.

The cognitive dysfunction may comprise, without limitation, the disease that is closely related to aging and, unlike the normal aging process, causes the death of abnormal nerve cells in a part of the nervous system or the entire brain rapidly, resulting in loss of function of the brain and spinal cord which decreases cognitive ability. A non-limiting example of the above cognitive dysfunction may comprise mild cognitive impairment, Alzheimer's disease, frontotemporal dementia, Lewy body disease, cortico-basal ganglia degeneration, learning disabilities, agnosia, amnesia, aphasia, apraxia and delirium.

In the composition of the present invention, the beta blocker and the cholinesterase inhibitor may be administered simultaneously, separately or sequentially, and the beta blocker and the cholinesterase inhibitor may exhibit brain disease prevention or treatment activity through a joint action in the body.

Typically, the beta blocker and the cholinesterase inhibitor will be administered simultaneously as a composition. However, even if each of the above-mentioned active ingredients is administered to the human body at different times, each individually administered active ingredient simultaneously acts in the body, thereby showing a level of therapeutic activity equal to that of simultaneous administration.

Specifically, the administration 'simultaneously' means administration of the two active ingredients together through the same route of administration, or administration via the same or different routes of administration, respectively, at substantially the same time (e.g., at the intervals of 15 minutes or less). The administration 'individually' means administration of the two active ingredients through the same or different routes of administration at a regular time interval (e.g., at the interval of 3 days). The administration 'sequentially' means administration of the two active ingredients through the same or different administration routes according to the patient's disease state with a certain ordering rule.

The route of administration may be oral or parenteral administration. The parenteral administration may comprise intravenous, intramuscular, intraarterial, intramedullary, intrathecal, intracardiac, transdermal, subcutaneous, intraneural, intraventricular (subventricular region), intracerebrovascular, intraperitoneal, intranasal, enteral, topical, sublingual or rectal administration, but the present invention is not limited thereto.

The pharmaceutical composition according to the present invention may comprise only pharmaceutically effective amounts of a beta blocker and a cholinesterase inhibitor, or may additionally comprise a pharmaceutically acceptable carrier. The 'pharmaceutically effective amount' refers to an amount that exhibits a higher response than that of the negative control, and, preferably, an amount sufficient to exhibit effects of increasing lifespan, improving motility, inhibiting neuroinflammation, inhibiting neuronal cell death, and promoting neuronal differentiation by administering the two active ingredients in combination in the treatment or prevention of brain diseases.

The pharmaceutical composition of the present invention, together with a pharmaceutically acceptable carrier, may be formulated in various ways according to the route of administration by a method known in the art to show the synergistic effect of combining the cholinesterase inhibitor and antioxidant. The term 'Pharmaceutically acceptable' refers to a non-toxic composition that is physiologically acceptable, does not inhibit the action of active ingredients when administered to a human being, and does not usually cause allergic reactions such as gastrointestinal disorders and dizziness or similar reactions. Such carriers comprise all kinds of solvents, dispersion media, oil-in-water or water-in-oil emulsions, aqueous compositions, liposomes, microbeads and microsomes. The pharmaceutically acceptable carrier contained in the composition is commonly used in formulation and comprises Lactose, Dextrose, Sucrose, Sorbitol, Mannitol, Starch, Gum Acacia, Calcium Phosphate, Alginate, Gelatin, Calcium Silicate, Microcrystalline Cellulose, Polyvinylpyrrolidone, Cellulose, Water, Syrup, Methyl Cellulose, Methylhydride comprising hydroxybenzoate, propylhydroxybenzoate, talc, magnesium stearate and mineral oil, but the present invention is not limited thereto. As other pharmaceutically acceptable carriers, reference may be made to those known in the art.

The pharmaceutical composition may further comprise a lubricant, a wetting agent, a sweetening agent, a flavoring agent, an emulsifying agent, a suspending agent, a preservative, and the like, in addition to the above-mentioned components. Specifically, for oral administeration, a binder, a lubricant, a disintegrant, an excipient, a solubilizer, a dispersant, a stabilizer, a suspending agent, a colorant or a flavoring agent may be used. In the case of an injection, a buffer, a preservative, an analgesic agent, a solubilizer, an isotonic agent or a stabilizer may be mixed and used. For topical administration, a base, an excipient, a lubricant or a preservative may be used.

In addition, the composition of the present invention may be used in the form of general pharmaceutical formulations. The formulations for parenteral administration may be prepared in sterilized aqueous solutions, non-aqueous solvents, suspensions, emulsions or lyophilized preparations, injections, transdermal preparations, or nasal inhalations. The formulations for oral administration may be prepared in the form of tablets, troches, capsules, elixirs, suspensions, syrups or wafers. Injections may be prepared in unit dose ampoules or multi-dose form. The injections must be sterilized and must be protected from contamination by microorganisms such as bacteria and fungi. For injections, examples of suitable carriers may be water, ethanol, polyols (e.g., glycerol, propylene glycol and liquid polyethylene glycol), mixtures thereof, and/or solvents or dispersion media comprising vegetable oils, but the present invention is not limited thereto. More preferably, suitable carriers comprise Hanks' solution, Ringer's solution, phosphate buffered saline (PBS) with triethanolamine or isotonic solutions such as sterile water for injection, 10% ethanol, 40% propylene glycol and 5% dextrose. In order to protect the injection from microbial contamination, various antibacterial and antifungal agents such as paraben, chlorobutanol, phenol, sorbic acid, and thimerosal may be further contained. Also, in most cases, the injection may further comprise an isotonic agent such as sugar or sodium chloride.

In addition, the pharmaceutical composition of the present invention may be administered by any device capable of moving an active substance to a target site. Preferred methods of administration and formulations are intravenous, subcutaneous, intradermal, intramuscular or intravenous injections. Injections may be prepared using aqueous solvents such as physiological saline or IV, vegetable oils, higher fatty acid esters (e.g., ethyl oleate), alcohols (e.g., ethanol, benzyl alcohol, propylene glycol and glycerin), and non-aqueous solvents, and may comprise pharmaceutical carriers which comprises a stabilizer to prevent deterioration (e.g., ascorbic acid, sodium hydrogen sulfite, sodium pyrosulfite, BHA, tocopherol and EDTA), an emulsifier, a buffer for pH control, and a preservative to prevent microbial growth (e.g., phenyl mercury nitrate, thimerosal, benzalkonium chloride, phenol, cresol and benzyl alcohol) . A method of treating or preventing neurodegenerative diseases using the composition of the present invention comprises administering an effective amount (pharmaceutically effective amount) of the composition for treatment of the present invention to a subject in need thereof. The pharmaceutically effective amount may be easily determined by a person skilled in the art according to factors well known in the medical field (e.g., type of disease, patient's age, weight, health, gender, patient's sensitivity to drugs, route of administration, method of administration, frequency of administration, duration of treatment, and drugs used in combination or concurrently).

In addition, the pharmaceutical composition of the present invention may be formulated using methods known in the art to provide rapid, sustained or delayed release of the active ingredient after administration to a mammal.

The total effective amount of the composition of the present invention may be administered to a patient in a single dose or by a fractionated treatment protocol in which multiple doses are administered over a long period of time. The pharmaceutical composition of the present invention may comprise an active ingredient whose content varies according to the severity of the disease. Preferably, the preferred total dose of the pharmaceutical composition of the present invention may be about 0.01 µg to 10,000 mg, most preferably 0.1 µg to 500 mg per kg of patient body weight per day. However, the effective dosage of the pharmaceutical composition for the patient is determined in consideration of the formulation method, administration route, number of treatments, and various factors (e.g., patient's age, weight, health status, gender, severity of disease, diet and excretion rate). Therefore, considering these points, those skilled in the art will be able to determine an appropriate effective dosage of the composition of the present invention. The pharmaceutical composition according to the present invention is not particularly limited in terms of its formulation, administration route and administration method, as long as it exhibits the effects of the present invention.

The composition of the present invention may be formulated such that a beta blocker and a cholinesterase inhibitor as components are simultaneously contained in one formulation, depending on the administration method and route. Each of the components may be individually formulated and contained in one package according to a dosage unit such as daily or once. The formulations of separately formulated beta blockers and cholinesterase inhibitors may be or may not be the same. The specific formulation method of the pharmaceutical composition of the present invention and the pharmaceutically acceptable carrier that may be contained in the formulation are as described above with regard to the pharmaceutical composition and are known in the art.

An aspect of the present invention is to provide a food composition comprising a beta blocker and a cholinesterase inhibitor for preventing or alleviating a neurodegenerative disease.

The food composition of the present invention comprises all forms such as functional food, nutritional supplement, health food and food additives, and is intended for consumption by animals such as a human being and livestock.

Food compositions of such type may be prepared in various forms according to conventional methods known in the art. As a general food, it can be prepared by adding the above-mentioned combination formulation to beverages (such as alcoholic beverages), fruits and their processed foods (e.g. canned fruits, bottled fruits, jams and marmalades), fish, meat and their processed foods (e.g. ham and sausage corned beef), breads and noodles (e.g. udon, buckwheat noodles, ramen, spagate and macaroni), fruit juice, various drinks, cookies, taffy, dairy products (e.g. butter and cheese), edible vegetable oil, margarine, vegetable protein, retort food, frozen food, or various seasonings (e.g. soybean paste, soy sauce and sauce), but the present invention is not limited thereto. In addition, nutritional supplements may be prepared by adding the above-mentioned combination formulations to capsules, tablets or pills, but the present invention is not limited thereto. In addition, health functional food may be ingested, for example, by liquefying, granulating, encapsulating, and powdering the combination formulation itself so that it may be prepared in the form of tea, juice, or drink and consumed (as a healthy beverage), but the present invention is not limited thereto. In addition, in order to use the combination formulation in the form of a food additive, it may be prepared and used in the form of a powder or concentrate. In addition, it can be prepared in the form of a composition by mixing the combination formulation with a known active ingredient commonly known in the art to have an effect on improving neurodegenerative diseases.

When the food composition of the present invention is used as a health beverage composition, the health beverage composition may comprise various flavoring agents or natural carbohydrates as additional components, like conventional beverages. The above-mentioned natural carbohydrates comprise monosaccharides such as glucose and fructose; disaccharides such as maltose and sucrose; polysaccharides such as dextrin and cyclodextrin; and a sugar alcohol such as xylitol, sorbitol and erythritol. The sweeteners may comprise natural sweeteners such as thaumatin and stevia extract; and synthetic sweeteners such as saccharin and aspartame. The proportion of the natural carbohydrate is generally about 0.01 to 0.04 g, preferably about 0.02 to 0.03 g per 100 mL of the composition of the present invention.

When the combination formulation according to the present invention is contained as an active ingredient in a food composition, the amount is not particularly limited to an amount effective to achieve symptom improvement, but is preferably 0.01 to 100% by weight based on the total weight of the total composition. The food composition of the present invention may be prepared by mixing the combination formulation with other active ingredients known to have an effect on improving neurodegenerative diseases.

In addition to the above, when used as a health food, the food composition of the present invention may comprise various nutrients, vitamins, electrolytes, flavors, colorants, pectic acid, salts of pectic acid, alginic acid, salts of alginic acid, organic acids, protective colloidal thickeners, pH adjusters, stabilizers, preservatives, glycerin, and alcohols or carbonating agents. In addition, the health food of the present invention may comprise fruit flesh for production of natural fruit juice, fruit juice beverage or vegetable beverage. These components may be used independently or in combination. The ratio of these additives is not very important, but is generally selected in the range of 0.01 to 0.1 parts by weight per 100 parts by weight of the composition of the present invention.

An aspect of the present invention is to provide use of a beta blocker and a cholinesterase inhibitor for the preparation of an agent for preventing or treating a neurodegenerative disease.

An aspect of the present invention is to provide a method for treating a neurodegenerative disease in a subject in need thereof, the method comprising administering an effective amount of a composition comprising a beta blocker and a cholinesterase inhibitor as active ingredients.

The term 'effective amount' of the present invention refers to an amount that, when administered to a subject, exhibits an effect of improving, treating, detecting, diagnosing, or suppressing or reducing neurodegenerative diseases or neurodegenerative diseases. The term 'individual' may be an animal, preferably a mammal, especially an animal comprising a human being, and may also be a cell, tissue or organ derived from an animal. The subject may be a patient in need of the effect.

The 'treatment' of the present invention comprehensively refers to improving neurodegenerative diseases or symptoms caused by the diseases, which may comprise curing, substantially preventing or ameliorating the condition, or alleviating, curing or preventing one or most of the symptoms resulting from the disease, but the present invention is not limited thereto.

As used herein, the term "comprising" is used in the same sense as "including" or "characterized by." The composition or method according to the present invention does not exclude additional components or method steps not specifically mentioned. In addition, the term "consisting of" refers to excluding additional elements, steps or components not separately described. The term "essentially consisting of" means that, in addition to the described materials or steps, materials or steps that do not substantially affect the basic characteristics thereof may be contained in the scope of a composition or method.

### Advantageous Effects of Invention

The composition according to the present invention can exhibit improved preventive and therapeutic effects of neurodegenerative diseases by co-administering a beta blocker and a cholinesterase inhibitor, as compared to administration of each of them, and has the effect of reducing side effects that may be caused by excessive administration or long-term administration of each drug.

### Brief Description of Drawings

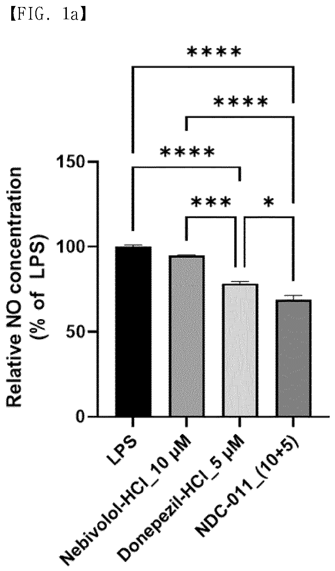

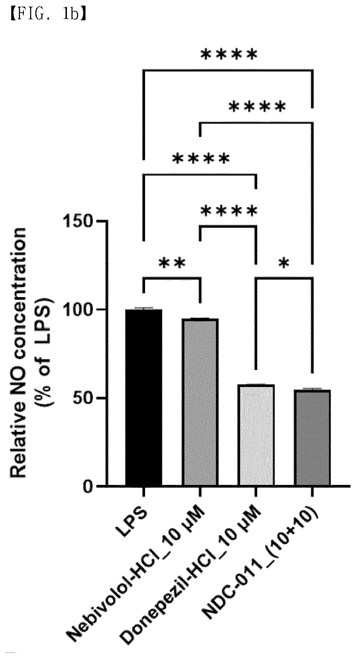

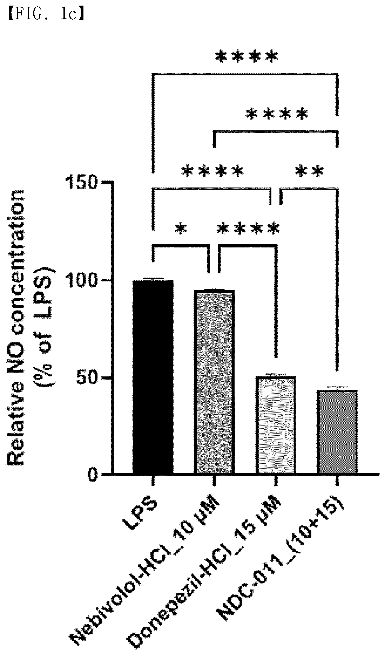

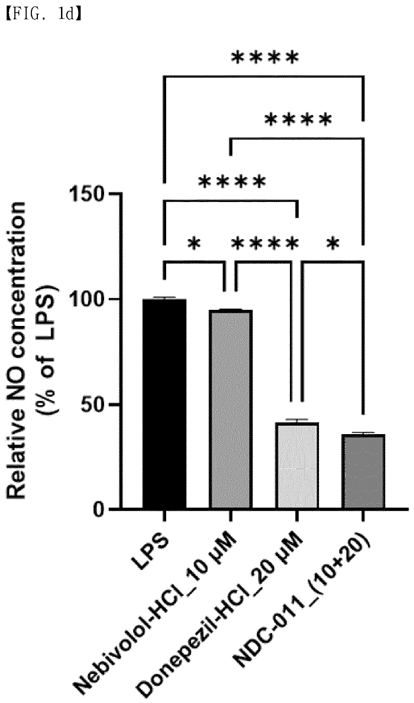

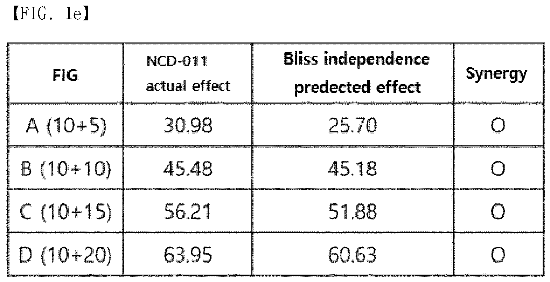

FIGS. 1A to 1E show the results of evaluating the anti-inflammatory effect by treating microglia (BV2 cells), together with lipopolysaccharide (LPS) as an inflammatory response inducer, with nebivolol alone, donepezil alone or nebivolol plus donepezil combination (NDC-011) and then measuring the generated NO concentration.

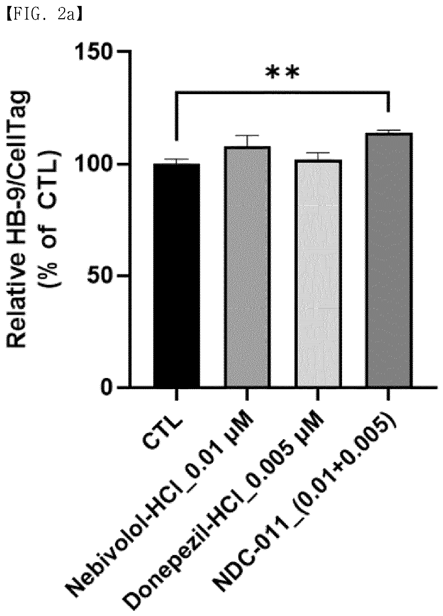

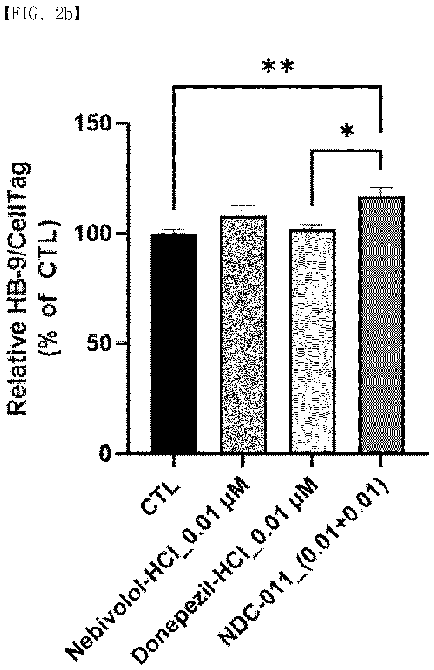

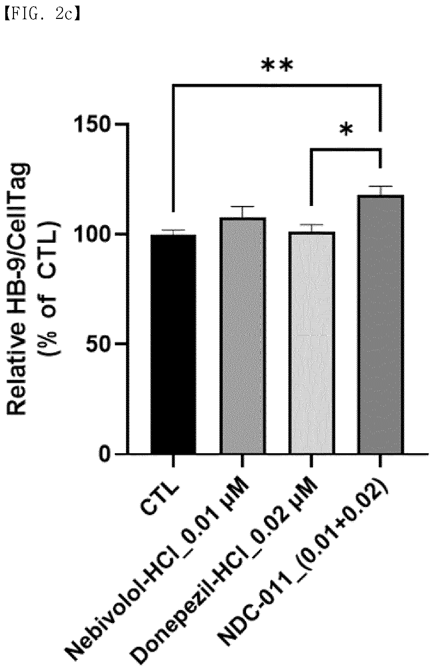

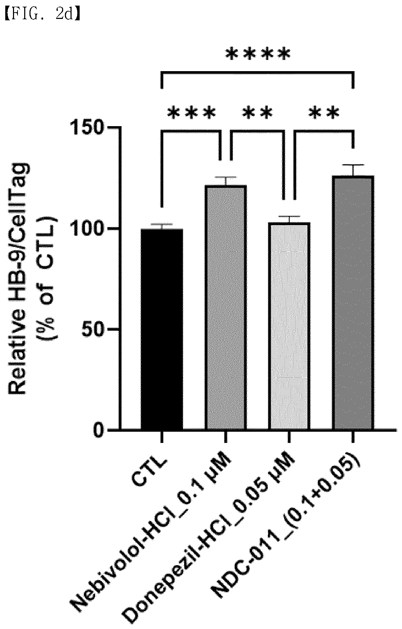

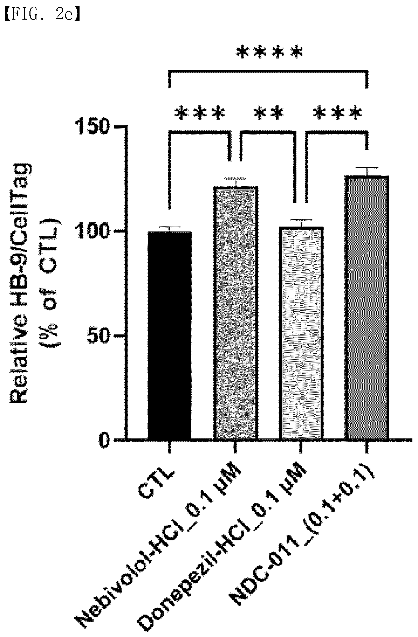

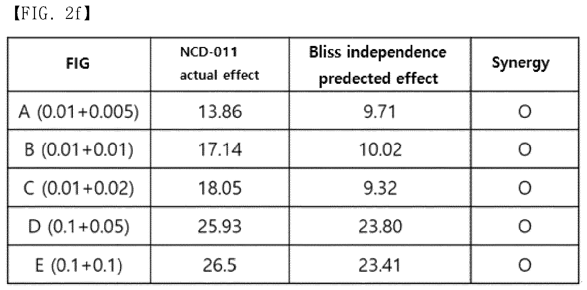

FIGS. 2A to 2F show the results of evaluating the differentiation promoting effect of a drug by culturing neural progenitor cells (ReNcell VM) under differentiation conditions treated with nebivolol alone, donepezil alone or the combination (NDC-011) and then evaluating the expression level of HB9 as a motor neuron marker.

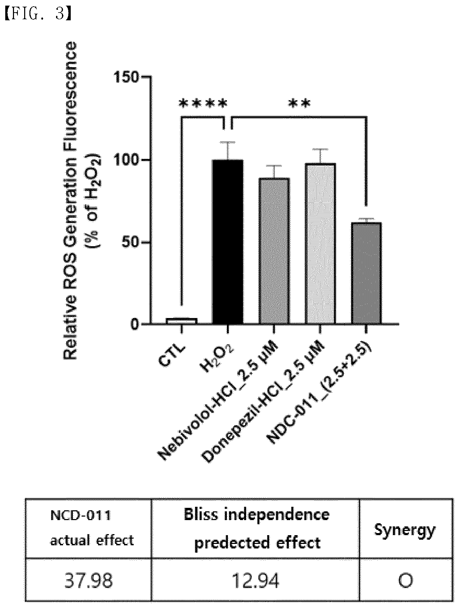

FIG. 3 shows the results of evaluating the antioxidant effect of a drug by treating neuroblasts (SH-SY5Y) with nebivolol alone, donepezil alone or the combination (NDC-011), inducing oxidative stress through hydrogen peroxide treatment, and measuring the amount of the generated reactive oxygen species (ROS).

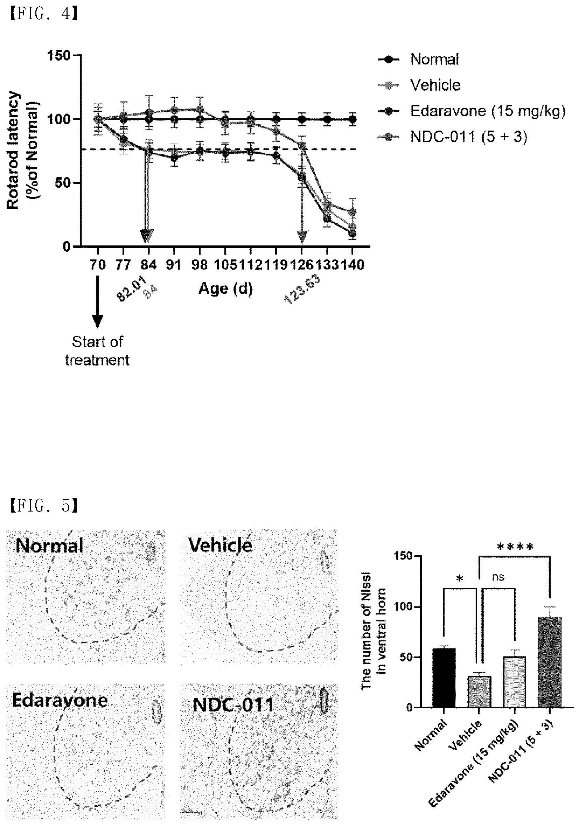

FIG. 4 shows the results of evaluating the animal's exercise capacity by orally administering edaravone or nebivolol + donepezil complex (NDC-011) to a 70-day-old ALS animal model for 70 days and performing Rotarod latency test at weekly intervals.
FIG. 5 shows the results of comparing the number of motor neurons in spinal cord tissue by Nissl staining by administering edaravone or nebivolol + donepezil combination (NDC-011) for 28 days to a 70-day-old ALS animal model and sampling the spinal cord tissue.

### Mode for Invention

Hereinafter, the present invention will be described in detail by the following embodiments. However, the following embodiments are only for illustrating the present invention, and the present invention is not limited thereto.

### Example 1: (In vitro) anti-inflammatory effect of nebivolol + donepezil complex

In the present application, microglia (BV2 cells) were treated with LPS to cause an inflammatory response and, at the same time, treated with nebivolol alone, donepezil alone, or nebivolol + donepezil combination to measure the concentration of generated nitrogen monoxide (NO), thereby confirming the effect of the drug on relieving neuroinflammation.

Microglia (BV2 cells) were treated with LPS to cause an inflammatory response and, at the same time, treated with nebivolol alone, donepezil alone, or nebivolol + donepezil combination to measure the concentration of generated NO. As a result, as shown in FIGS. 1A to 1E, it was confirmed that the group treated with the drug showed a significant reduction in NO, as compared to the LPS control group, and the reduction effect was greatest in the group treated with nebivolol + donepezil combination. In addition, it was confirmed that, when calculated by applying the Bliss independence model, the effect of the treatment with a combination of the two drugs was higher than that expected from the treatment with each of the two drugs, thereby confirming the synergistic effect of the two drugs. (**P* < 0.05, ***P* < 0.01, *** *P* < 0.001, **** *P* < 0.0001, one-way ANOVA)

### Example 2: (In vitro) effect of nebivolol + donepezil combination on promoting differentiation of neural progenitor cells

Immortalized human neural progenitor cell line (ReNcell VM) as a neural progenitor cell that may differentiate into neurons and glial cells was treated with nebivolol alone, donepezil alone, and nebivolol + donepezil combination. Thereafter, immunostaining was performed using an antibody capable of selectively binding to motor neurons (anti-HB9 antibody) and a CellTag probe that stains whole cells. Thereafter, the HB9 positive signal and the value of CellTag were analyzed to determine the number of motor neurons among the total cells, thereby measuring the degree of differentiation of neural precursor cells into motor neurons.

As a result, as shown in FIGS. 2A to 2F, it was confirmed that the groups treated with nebivolol alone and nebivolol + donepezil combination showed a significant increase in HB-9 expression, as compared to the control group. Such increase was not observed in the group treated with a low concentration (0.01 pM) of nebivolol or donepezil alone. In addition, the prediction results through the Bliss independence model corroborated that the effect of the treatment with the combination was greater than the expected effect, thereby confirming that nebivolol and donepezil as drugs showed a synergistic effect in promoting the differentiation of neural progenitor cells. (**P* < 0.05, ***P* < 0.01, *** *P* < 0.001, **** *P* < 0.0001, one-way ANOVA)

### Example 3: (In vitro) antioxidant effect of nebivolol + donepezil combination

In the present application, neuroblasts (SH-SY5Y) were treated with nebivolol alone, donepezil alone, or nebivolol + donepezil combination, and treated with dichlorofluores (DCF) which emits a fluorescent signal in proportion to the amount of reactive oxygen species, and H₂O₂ as an oxidative stress inducer to measure the amount of the generated reactive oxygen species (ROS), thereby confirming the effect of the drug on reducing the generation of reactive oxygen species.

As a result, as shown in FIG. 3, only the group treated with the combination showed a significant decrease in ROS, as compared to the control group, and no significant decrease was observed in the group administered with either nebivolol or donepezil alone. In addition, the prediction results through the Bliss independence model corroborated that the effect of the treatment with the combination was greater than the expected effect, thereby confirming that the combined use of nebivolol + donepezil showed a synergistic effect in antioxidation. (**P<0.01, ****P<0.0001, one-way ANOVA)

### Example 4: In vivo ALS treatment effect

SOD1G93A transgenic mice as ALS animal models were used to verify the in vivo efficacy of the nebivolol and donepezil complex. Edaravone (15 mg/kg) as a commercially available ALS treatment, or a nebivolol and donepezil complex (NDC-011, nebivolol 5 mg/kg + donepezil 3 mg/kg) according to the present invention was administered to mice from 70 days of age once daily. Each dose was orally administered for 70 days. The rotarod latency test was performed immediately before the start of drug administration to evaluate the motor ability of each animal, and, thereafter, the rotarod latency test was performed during the drug administration period at 7-day intervals to evaluate the motor ability of the ALS animals. As a result, the placebo-treated group or the group treated with edaravone, as a commercially available treatment, showed a significant decrease in exercise capacity from the age of 84 days, as compared to the normal control group. On the other hand, the group administered with the complex according to the present invention showed a decrease in exercise capacity from 123 days of age. As a result, it was confirmed that the NDC-011 complex delayed the period when the motility of ALS animals decreased, by about 40 days (see FIG. 4).

Edaravone or the NDC-011 combination drug (nebivolol 5 mg/kg + donepezil 3 mg/kg) was started to be administered to a 70-day-old ALS animal models and then administered daily for 1 each dose for 28 days, in order to confirm the inhibitory effect of the NDC-011 combination drug on motor neuron cell death. Thereafter, the spinal cord tissue of the TG mouse was sampled, motor neurons were stained with Nissl staining, and the number was confirmed. As a result, the placebo-treated group showed a significantly decrease in the number of motor neurons, as compared to the normal control group, which confirmed that motor neuron cell death occurred in the ALS animal model. In addition, it was confirmed that the group administered with nebivolol and donepezil complex (NDC-011) showed a significant increase in in the number of motor neurons, as compared to the group administered with placebo or edaravone, and also showed an increase in the number, as compared to the normal control group. Therefore, the effects of the NDC-011 complex on inhibiting motor neuron cell death and increasing the number of motor neuron cells in the ALS animal models were confirmed (see FIG. 5) .

### Industrial Applicability

The composition according to the present invention can exhibit increased preventive and therapeutic effects on neurodegenerative diseases by co-administering a beta blocker and a cholinesterase inhibitor, as compared to administration of each of them, and has an effect of reducing side effects that may be caused by excessive or long-term administration of each drug and, thus, can be very usefully used in the development of a treatment for neurodegenerative diseases.

## Claims

1. A pharmaceutical composition comprising a beta blocker and a cholinesterase inhibitor as active ingredients for preventing or treating a neurodegenerative disease.

2. The pharmaceutical composition of claim 1, wherein the neurodegenerative disease is selected from the group consisting of Alzheimer's disease, Parkinson's disease, dementia, cognitive dysfunction, progressive supranuclear palsy, multiple system atrophy, olive-ponytic-cerebellar atrophy (OPCA), Shy-Drager syndrome, striatal-substantia nigra degeneration, Huntington's disease, amyotrophic lateral sclerosis (ALS), essential tremor, cortico-basal ganglia, diffuse Lewy body disease, Parkinson-ALS-dementia complex, Niemann-Pick disease and Pick's disease.

3. The pharmaceutical composition of claim 1, wherein the beta blocker is selected from the group consisting of Nebivolol, Acebutolol, Alprenolol, Atenolol, Betaxolol, Bisoprolol, Bucindolol, Carteolol, Carvedilol, Celiprolol, Esmolol, Labetalol, Levobunolol, Medroxalol, Mepindolol, Metipranolol, Metoprolol, Nadolol, Oxprenolol, Penbutolol, Pindolol, Propranolol, Sotalol, Timolol and salts thereof.

4. The pharmaceutical composition of claim 1, wherein the cholinesterase inhibitor is selected from the group consisting of donepezil, 7-methoxytacrine, albameline, ambenonium, anseculin, arecoline, cevimeline, citicoline, demacarium, donepizil, edrophonium, eptastigmine, fasciculin, heptyl-physostigmine, huperzine A and its analogs, icopezil, ipidacrine, linopiridine, metrifonate, milameline, neostigmine, nomeostigmine, pyridostigmine, norpyridostigmine, tacrine, physostigmine, rivastigmine, subcomeline, suronacrine, tacrine analogs, tacrine, talsaclidine, velnacrine, xanomeline, ziprosilone, itopride, acotiamide , huperzine, galantamine, ziprosilone, and salts thereof.

5. The pharmaceutical composition of claim 1, wherein the composition exhibits the effect of alleviating neuroinflammation, promoting neuronal differentiation and anti-oxidation.

6. The pharmaceutical composition of claim 1, wherein the beta blocker and the cholinesterase inhibitor are co-administered in a molar ratio of 1:0.1 to 20.

7. The pharmaceutical composition of claim 1, wherein the beta blocker and the cholinesterase inhibitor are co-administered simultaneously, separately or sequentially.

8. The pharmaceutical composition of claim 1, wherein the beta blocker is nebivolol represented by Formula I or a pharmaceutically acceptable salt thereof:

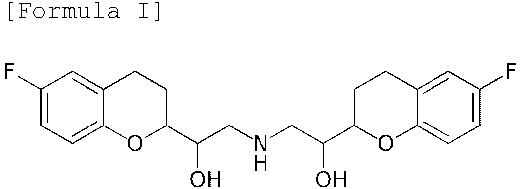

9. The pharmaceutical composition of claim 1, wherein the cholinesterase inhibitor is donepezil represented by formula II or a pharmaceutically acceptable salt thereof:

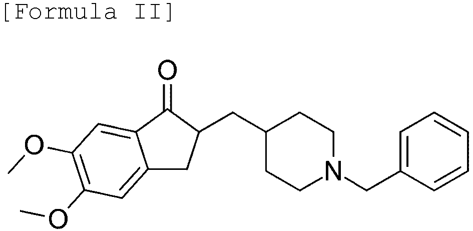

10. A food composition comprising a beta blocker and a cholinesterase inhibitor for preventing or alleviating a neurodegenerative disease.

11. The food composition of claim 10, wherein the food is a functional food.

12. Use of a beta blocker and a cholinesterase inhibitor for the preparation of an agent for preventing or treating a neurodegenerative disease.

13. A method for treating a neurodegenerative disease in a subject in need thereof, the method comprising administering an effective amount of a composition comprising a beta blocker and a cholinesterase inhibitor as active ingredients.
